# FASCICULE DE BREVET EUROPEEN

(11) **EP 2 459 013 B1**
(45) Date de publication et mention de la délivrance du brevet: **18.12.2013**
(21) Numéro de dépôt: 10736717.9
(22) Date de dépôt: 26.07.2010
(51) Int. Cl.: A23L 1/30, A61K 36/00

(54) **COMPOSITION NUTRITIVE POUR FEMMES ALLAITANT**
NAHRUNGSMITTEL FÜR STILLENDEN FRAUEN
FOOD COMPOSITION FOR LACTATING WOMEN

(30) Priorité: 29.07.2009 FR 0955301
(43) Date de publication de la demande: 06.06.2012
(73) Titulaire: Laboratoires France Bebe Nutrition, 53000 Laval (FR)
(72) Inventeur: TEK, Konthirith, F-53000 Laval (FR); KERRAND, Solenn, F-53000 Laval (FR); GIORDANO, Thierry, F-53000 Laval (FR)
(74) Mandataire: Maillet, Alain
(86) Numéro de dépôt international: PCT/EP2010/060778
(87) Numéro de publication internationale: WO 2011/012568

(56) Documents cités:
- EP-A2- 0 705 539
- US-A- 4 710 837
- US-A1- 2001 024 665
- US-A1- 2002 136 783
- DATABASE WPI Week 199929 Thomson Scientific, London, GB; AN 1999-340388 XP002569595 KASS CD: "Natural lactation stimulant - prepared by extracting a mixture containing various plants e.g., coriander" & IL 109 916 A (KASS C D) 11 avril 1999 (1999-04-11)
- DATABASE WPI Week 200631 Thomson Scientific, London, GB; AN 2006-296247 XP002569596 "Herbal tea to stimulate lactation" & HU 0 303 209 A1 (NAGY L) 29 août 2005 (2005-08-29)
- DATABASE WPI Week 199716 Thomson Scientific, London, GB; AN 1997-166370 XP002569597 WU Y: "Lactifuge for delactation of women" & CN 1 092 664 A (WU Y) 28 septembre 1994 (1994-09-28)
- WPI WORLD PATENT INFORMATION DERWENT, vol. 22, no. 91, 159781, 19 avril 1991 (1991-04-19), XP002020289

## Description

La présente invention concerne une composition nutritive pour femmes allaitant leur enfant.

Durant l'allaitement, les femmes présentent des besoins spécifiques en certains nutriments et devraient, de ce fait, recevoir un régime alimentaire particulier. En effet, l'alimentation de la femme au cours de cette période doit non seulement couvrir ses propres besoins mais également ceux du nourrisson.

Les besoins de la femme allaitant sont soit liés à des déficiences en certains éléments indispensables observées à la fin de la grossesse, soit liés au transfert des nutriments de la mère à l'enfant durant l'allaitement, soit liés aux besoins propres au développement physiologique et neurologique de l'enfant. Par exemple, on observe à la fin de la grossesse des carences en certaines vitamines, notamment en vitamine D. Les acides gras, tels que les acides gras du groupe des Omégas 3, notamment l'acide docosahexaénoïque (DHA), sont indispensables au développement du cerveau pendant la période foetale et la petite enfance et devraient être présents dans l'alimentation de la mère. Les besoins énergétiques, en protéines, en lipides ou en glucides sont très augmentés chez la femme qui allaite comparativement à celle qui n'allaite pas, une partie de ces nutriments étant absorbés par l'enfant.

Malheureusement les femmes qui allaitent ne trouvent pas nécessairement, ou n'intègrent pas, de quoi combler ces besoins dans leur alimentation. Pour pallier ces problèmes, il est souvent nécessaire d'avoir recours à des suppléments nutritionnels, comme par exemple décrits dans EP 0705539 et US 4710387.

Par ailleurs, l'allaitement d'un nourrisson par sa mère nécessite une qualité de la sécrétion lactée. Chez les femmes qui allaitent, il est parfois nécessaire d'aider la femme à maintenir cette qualité des sécrétions lactées.

La sécrétion lactée est le résultat d'un contrôle endocrine exercé principalement par deux hormones: la prolactine, en ce qui concerne la sécrétion du lait, et l'ocytocine, en ce qui concerne l'éjection. L'insuffisance de sécrétion lactée connaît des causes multiples dont notamment un état de stress et de fatigue de la mère. Il est connu, par exemple, que la libération d'ocytocine peut être influencée par de nombreux facteurs tels que le stress dont le rôle inhibiteur est bien connu. Les sécrétions de prolactine par l'hypophyse peuvent quant à elles être déclenchées par la succion.

L'invention se propose de résoudre l'ensemble des problèmes sus mentionnés. Le but de la présente invention est ainsi de proposer une composition nutritive unique pour femmes allaitant qui répond à la fois à ses propres besoins nutritionnels et à ceux du nouveau-né et qui favorise, de plus, l'allaitement.

A cet effet, l'invention concerne une composition nutritive comprenant une fraction protéique, une fraction glucidique, une fraction lipidique comprenant au moins un acide gras du groupe des omégas 3, au moins un élément minéral, au moins une vitamine dont la vitamine D et une combinaison d'extraits de plantes choisie parmi le malt-orge (*Hordeum vulgare*) et le fenugrec (*Trigonella foenum graecum*), ou le malt-orge et l'anis vert (*Pimpinella anisum*), ou le fenugrec et l'anis vert, ou encore le malt-orge, le fenugrec et l'anis vert.

Les plantes sont aujourd'hui très souvent associées à l'alimentation humaine ou animale en raison de leurs nombreuses vertus. Elles présentent en effet des propriétés physiologiques intéressantes chez l'homme, ou l'animal, et leur exploitation dans des compositions nutritives, des compléments alimentaires ou des compositions pharmaceutiques est aujourd'hui bien accueillie.

Les plantes appartenant au groupe formé par le houblon, le malt-orge, le fenugrec, l'anis vert, le fenouil, le cumin, le basilic, l'ortie blanche, le niaouli, le carvi, la pensée sauvage, le quinoa et le galega présentent une activité favorisant la lactation chez la femme, c'est-à-dire favorisant l'initiation, la production et le maintien de la sécrétion lactée et, plus généralement, favorisant l'allaitement. A titre d'exemple, l'orge est utilisée traditionnellement pour stimuler la sécrétion de prolactine et favoriser ainsi la lactation. L'anis vert présente une activité "oestrogène-like", c'est à dire qu'elle exerce une fonction hormonale similaire à celle des oestrogènes, lesquelles jouent un rôle au cours de l'initiation de la lactation. Par conséquent, ces plantes possèdent des propriétés galactagogues intéressantes qui permettent de stimuler la sécrétion lactée. Dans le cadre de l'invention sont utilisées les combinaisons suivantes d'extraits: malt-orge et fenugrec, malt-orge et anis vert, fenugrec et anis vert, ou encore malt-orge, fenugrec, et anis vert.

La composition selon l'invention contient ainsi des protéines, des lipides, des glucides ainsi que des vitamines et des minéraux qui sont nécessaires aux besoins de la femme allaitant et à l'enfant se nourrissant de son lait. Elle présente l'avantage supplémentaire de contenir un ou plusieurs extraits de plantes permettant de favoriser la lactation et, plus généralement, de favoriser l'allaitement. Cette composition est ainsi parfaitement adaptée à la condition de la femme en période d'allaitement.

La fraction protéique peut être d'origine variée. Il peut s'agir de protéines d'origine animale, végétale, laitière ou un mélange. La source de protéines peut être, par exemple, constituée par un lait entier, demi-écrémé ou écrémé sous forme liquide ou sous forme de concentré.

La fraction lipidique peut être n'importe quelle fraction usuellement utilisée pour la préparation de formule infantile. Elle peut être obtenue à partir d'huile ou de graisse végétale ou animale, laitière, à partir d'oeufs ou encore à partir d'huile de poisson. De même, toute source de glucides peut être envisagée.

La composition selon l'invention inclut dans sa fraction lipidique au moins un acide gras dit essentiel du groupe des oméga-3. Les acides gras du groupe des oméga-3 sont par exemple l'acide eicosapentaénoique (EPA), l'acide docosahexaénoique (DHA) l'acide alpha linolénique (ALA) et les acides gras du groupe des oméga-6 sont par exemple l'acide linoléique (LA) et l'acide arachidonique (AA). Une combinaison d'un ou de plusieurs acides gras appartenant aux groupes des oméga-3 et des oméga-6 est également envisagée selon l'invention. Ces acides gras essentiels, en particulier les oméga-3, sont indispensables à la croissance et au développement du cerveau pendant la période néonatale.

Selon un mode préféré de réalisation de l'invention, l'acide gras du groupe des omégas 3 dans la fraction lipidique est l'acide docosahexaénoïque ou DHA.

La vitamine D trouve son importance dans la composition selon l'invention car elle permet de pallier les carences chez la femme à la fin de la grossesse et participe également à la fixation du calcium sur les os du squelette du nouveau-né. Elle est apportée sous une forme naturelle telle que l'ergocalciférol ou le cholécalciférol.

Le, ou les, composé(s) choisi(s) parmi les autres vitamines et les minéraux peuvent être, par exemple, en ce qui concerne les vitamines, de la vitamine A, de la vitamine B1, de la vitamine B2, de la vitamine B3, de la vitamine B6, de la vitamine B9, de la vitamine B12, de la vitamine E, de la vitamine C, en ce qui concerne les minéraux, le sélénium, le calcium, le phosphore, l'iode, le magnésium, le zinc.

Les vitamines et les minéraux sont utilisés dans la composition à des teneurs recommandées pour la femme allaitant.

Selon un mode de réalisation de l'invention, la composition comprend de l'iode, du sélénium, du magnésium.

Une partie de l'iode présente dans l'alimentation de la mère est absorbée par le nourrisson via le lait maternel. La composition selon l'invention permet de subvenir aux besoins de la mère et de l'enfant. L'iode est présent dans la composition principalement sous forme d'iodure de potassium ou de toute autre forme autorisée.

Le sélénium est apporté sous forme de sélénite ou de toute autre forme autorisée.

Le magnésium est apporté sous forme de chlorure de magnésium ou de toute autre forme autorisée.

Selon un mode de réalisation de l'invention, la composition comprend une base laitière, préférentiellement choisie parmi un lait entier, demi-écrémé ou écrémé sous forme liquide ou sous forme de concentré.

Le produit laitier permet, entre autres, d'apporter le calcium dans l'alimentation de la mère, lequel participe à la construction du squelette du nouveau-né.

Préférentiellement, le lait est sous une forme liquide. Ce mode de réalisation permet avantageusement de couvrir en partie les apports hydriques de la femme allaitant, ainsi que fortement recommandé par le corps médical.

Selon ce mode particulier de réalisation, les extraits de plantes, les lipides, les glucides, les minéraux et les vitamines sont ajoutés lors du procédé de fabrication du lait consommable entre les étapes de standardisation et de stérilisation comme l'illustreront les exemples qui suivront.

Parmi les combinaisons d'extraits de plantes envisagées dans la présente invention, selon un mode préféré de réalisation, celle-ci comprend un extrait de plante de malt-orge, un extrait de fenugrec et un extrait d'anis vert.

Cette combinaison d'extraits de plante dans la composition est particulièrement avantageuse. En effet, le malt-orge présente l'avantage de stimuler la sécrétion de prolactine, l'anis vert présente une activité oestrogénique et, enfin, le fenugrec permet de modifier favorablement le goût du lait produit par la femme et stimule ainsi la succion. Les extraits de plantes exercent alors un effet de synergie favorisant l'allaitement en initiant, stimulant et en maintenant le phénomène de lactation.

Selon un autre mode préféré de réalisation de l'invention, la composition comprend un extrait de plante de malt-orge et un extrait de fenugrec ou un extrait de malt-orge et un extrait d'anis vert ou un extrait de fenugrec et un extrait d'anis vert.

Selon un mode de réalisation de l'invention, la composition comprend une source de fibres, préférentiellement choisie parmi le galactofructose et/ou les fructooligosaccharides. Les fibres ont pour but d'aider le fonctionnement du transit intestinal de la femme. De plus le galactofructose et les fructooligosaccharides sont des composés prébiotiques qui stimulent la croissance et l'activité métabolique de bactéries non pathogènes de la flore intestinale.

Selon un mode de réalisation préféré de l'invention, la composition nutritive apporte quotidiennement:
- entre 0, 1 µg et 10 µg, en particulier entre 1 et 2 µg deVitamine D,
- entre 100 mg et 1 g, en particulier entre 200 et 300 mg de calcium,
- entre 1 et 100 µg, en particulier entre 10 et 30 µg de sélénium,
- entre 10 et 200 µg, en particulier entre 30 et 60 µg d'iode
- entre 10 et 400 mg, en particulier entre 110 et 130 mg de magnésium,
- entre 100 et 1000 mg, en particulier entre 230 et 250 mg de phosphore,
- entre 0,1 et 30 mg de fibres,
- entre 10 et 500 mg, en particulier entre 50 et 150mg d'acide docosahexaénoïque,
- entre 0,01 et 2 g d'acides gras polyinsaturés à longue chaîne,
- entre 10 et 6000 mg d'une combinaison d'extraits de plantes choisie parmi le malt-orge *(Hordeum vulgare)* et le fenugrec (*Trigonella foenum graecum*), ou le malt-orge et l'anis vert (*Pimpinella anisum*), ou le fenugrec et l'anis vert, ou encore le malt-orge, le fenugrec et l'anis vert.

Dans une telle composition, les lipides représentent 20 à 40 % de l'apport calorique de la composition, les protéines représentent 10 à 30 % de l'apport calorique de la composition, les glucides représentent 40 à 60 % de l'apport calorique de la composition.

L'apport quotidien pourra par exemple être réalisé par le biais d'une formulation unique de 100, de 200 mL, ou plus. Cet apport peut également être réparti en différentes doses sur une journée.

Selon un mode particulier de réalisation de l'invention, la composition apporte quotidiennement:
- de 10 à 300 mg, en particulier entre 170 et 210 mg, de malt d'orge,
- de 50 à 2000 mg, en particulier entre 50 et 150 mg de fenugrec,
- de 100 à 4000 mg, en particulier entre 100 et 200 mg, d'anis vert. Avantageusement, la composition se présente sous la forme d'un liquide, d'une poudre, d'un aliment solide ou semi-solide, d'un comprimé, d'un granulé, d'une solution contenue dans une ampoule ou de toute autre forme galénique appropriée.

L'invention concerne encore une composition telle que définie précédemment pour son utilisation en tant que médicament.

L'invention concerne encore une composition telle que définie précédemment pour favoriser l'allaitement, en particulier pour stimuler et/ou initier la lactation et/ou stimuler la succion durant l'allaitement chez un mammifère, en particulier chez l'homme, ou encore l'utilisation d'une composition telle que définie précédemment pour la préparation d'un produit destiné à favoriser l'allaitement, en particulier stimuler et/ou initier la lactation et/ou stimuler la succion durant l'allaitement chez un mammifère, en particulier chez l'homme.

Enfin, l'invention concerne une méthode pour favoriser l'allaitement, en particulier pour stimuler et/ou initier la lactation et/ou stimuler par un nourrisson durant l'allaitement, comprenant l'administration à une femme allaitant d'une composition telle que définie précédemment chez un mammifère, en particulier chez l'homme.

La composition est généralement ingérée une fois par jour, préférentiellement sous la forme d'une dose unique.

L'invention est illustrée dans les exemples qui suivent qui se veulent illustratifs et non limitatifs.

### Composition nutritive pour femme allaitant se présentant sous forme d'un lait liquide prêt à consommer:

### Quantité/100 ml reconstituée:

Vitamine D : 0,75 µg
Calcium : 120 mg
Sélénium : 8,25 µg
Iode : 22,5 µg
Magnésium : 56,25 mg
Phosphore : 120 mg
Fibres : 0,1 g
Galactofructose : 1,26g
Lipides : 1,9g
   Dont DHA : 40 mg
Protéines : 3,9 g
Glucides : 6,4 g
   dont sucres: 6,4 g
Malt d'orge de 0,01 à 0,1 g
Fenugrec de 0,01 à 0,1 g
Anis vert de 0,01 à 0,1 g

### Préparation:

- Standardisation du lait stérilisé UHT: écrémage du lait en fonction du type de lait recherché: lait entier (35 g/l de matières grasses; lait demi-écrémé (15-18 g/l de MG); lait écrémé (maximum 3 g/l de MG) ;
- ajout des vitamines, des minéraux, des fibres, des oméga-3 et des extraits de plantes ;
- Préchauffage à 70-75°C ;
- Homogénéisation ;
- Stérilisation (135-150°C pendant 2 à 6 secondes) par chauffage indirect sur un échangeur tubulaire ;
- Refroidissement ;
- Conditionnement sous atmosphère aseptique et emballages stériles.

## Revendications

1. Composition nutritive comprenant :
- une fraction protéique,
- une fraction glucidique,
- une fraction lipidique comprenant au moins un acide gras du groupe des omégas 3,
- au moins un élément minéral,
- au moins une vitamine dont la vitamine D,
- une combinaison d'extraits de plantes choisie parmi le malt-orge (*Hordeum vulgare*) et le fenugrec (*Trigonella foenum graecum*), ou le malt-orge (*Hordeum vulgare*) et l'anis vert (*Pimpinella anisum*), ou le fenugrec (*Trigonella foenum graecum*) et l'anis vert (*Pimpinella anisum*), ou encore le malt-orge (*Hordeum vulgare*), le fenugrec (*Trigonella foenum graecum*) et l'anis vert (*Pimpinella anisum*).

2. Composition selon la revendication 1, **caractérisée en ce qu'**elle comprend une base laitière, préférentiellement choisie parmi un lait liquide ou un concentré de lait séché.

3. Composition selon l'une des revendications 1 à 2, **caractérisée en ce que** ledit acide gras du groupe des omégas 3 est l'acide docosahexaénoïque ou DHA.

4. Composition selon l'une des revendications 1 à 3, **caractérisée en ce que** qu'elle comprend de l'iode, du sélénium et du magnésium parmi les minéraux.

5. Composition selon l'une des revendications 1 à 4, **caractérisée en ce qu'**elle comprend, de plus, une source de fibres, préférentiellement choisie parmi le galactofructose et/ou le fructooligosaccharide.

6. Composition selon l'une des revendications 1 à 5, **caractérisée en ce qu'**elle se présente sous la forme d'un liquide, d'une poudre, d'un aliment, d'un comprimé, d'un granulé, d'une solution contenue dans une ampoule ou de toute autre forme galénique appropriée.

7. Composition telle que définie dans l'une des revendications 1 à 6 pour son utilisation en tant que médicament.

8. Dose d'une composition telle que définie dans l'une des revendications 1 à 7, **caractérisée en ce qu'**elle est formée de:
Vitamine D : de 0, 1 µg à 10µg, en particulier de 1 à 2 µg,
Calcium : 100mg à 1 g, en particulier de 200 à 300 mg,
Sélénium : de 1 à 100 µg, en particulier de 10 à 30 µg,
Iode : 10 à 200 µg, en particulier de 30 à 60 µg,
Magnésium : 10 à 400 mg, en particulier de 110 à 130 mg,
Phosphore : 100 à 1000 mg, en particulier de 230 à 250 mg,
Fibres : 0,1 à 30 mg,
Acide docosahexaénoïque : 10 à 500 mg, en particulier de 50 à 150mg,
Acides gras polyinsaturés à longue chaîne : 0,01 à 2 g,
entre 10 et 6000 mg, d'une combinaison d'extraits de plantes choisie parmi le malt-orge et le fenugrec ou le malt-orge et l'anis vert ou le fenugrec et l'anis vert ou encore le malt-orge, le fenugrec et l'anis vert.

9. Dose selon la revendication 8, **caractérisée en ce qu'**elle comprend entre 10 et 300 mg, en particulier entre 170 et 210 mg de Malt d'orge, entre 50 et 2000 mg, en particulier entre 50 à 150 mg de Fenugrec et entre 100 et 4000 mg, en particulier entre 100 et 200 mg d'Anis vert.

10. Composition telle que définie dans l'une des revendications 1 à 7 pour son utilisation pour stimuler et/ou initier la lactation et/ou stimuler la succion durant l'allaitement chez un mammifère, en particulier chez l'homme.

11. Dose telle que définie dans l'une des revendications 8 à 9 pour son utilisation pour stimuler et/ou initier la lactation et/ou stimuler la succion durant l'allaitement chez un mammifère, en particulier chez l'homme.

## Patentansprüche

1. Nahrungszusammensetzung enthaltend
- eine Proteinfraktion,
- eine Kohlenhydratfraktion,
- eine Fettfraktion enthaltend mindestens eine Fettsäure aus der Gruppe der Omega-3-Fettsäuren,
- mindestens einen mineralischen Bestandteil,
- mindestens ein Vitamin, darunter Vitamin D,
- eine Kombination von Pflanzenextrakten aus Gerstenmalz (*Hordeum vulgare*) und Bockshornklee (*Trigonella foenum graecum*) oder Gerstenmalz (*Hordeum vulgare*) und grünem Anis (*Pimpinella anisum*) oder Bockshornklee (*Trigonella foenum graecum*) und grünem Anis (*Pimpinella anisum*) oder zusätzlich Gerstenmalz (*Hordeum vulgare*), Bockshornklee (*Trigonella foenum graecum*) und grünem Anis (*Pimpinella anisum*).

2. Zusammensetzung nach Anspruch 1,
**dadurch gekennzeichnet, dass**
sie eine vorzugsweise aus flüssiger Milch oder einem Konzentrat aus Trockenmilch ausgewählte Milchgrundlage umfasst.

3. Zusammensetzung nach Ansprüchen 1 und 2,
**dadurch gekennzeichnet, dass**
die Fettsäure aus der Omega 3-Gruppe Docosahexaensäure oder DHA ist.

4. Zusammensetzung nach einem der Ansprüche 1 bis 3,
**dadurch gekennzeichnet, dass**
sie lod, Selen und Magnesium als Mineralien umfasst.

5. Zusammensetzung nach einem der Ansprüche 1 bis 4,
**dadurch gekennzeichnet, dass**
sie außerdem eine vorzugsweise aus Lactulose und/oder Oligofructose ausgewählte Ballaststoffquelle umfasst.

6. Zusammensetzung nach einem der Ansprüche 1 bis 5,
**dadurch gekennzeichnet, dass**
sie in Form einer Flüssigkeit, eines Pulvers, eines Nahrungsmittels, einer Tablette, eines Granulats, einer in einer Ampulle enthaltenen Lösung oder in allen weiteren galenisch geeigneten Formen vorliegt.

7. Zusammensetzung nach einem der Ansprüche 1 bis 6 zur Verwendung als Medikament.

8. Dosis der wie in einem der Ansprüche 1 bis 7 definierten Zusammensetzung, **dadurch gekennzeichnet, dass**
sie aus:
Vitamin D: von 0,1 µg bis 10 µg, insbesondere 1 bis 2 µg,
Calcium: 100 mg bis 1 g, insbesondere 200 bis 300 mg,
Selen: von 1 bis 100 µg, insbesondere 10 bis 30 µg,
Iod:10 bis 200 µg, insbesondere 30 bis 60 µg,
Magnesium: 10 bis 400 mg, insbesondere 110 bis 130 mg,
Phosphor: 100 bis 1000 mg, insbesondere von 230 bis 250 mg,
Ballaststoffe: 0,1 bis 30 mg,
Docosahexaensäure: 10 bis 500 mg, insbesondere 50 bis 150 mg,
mehrfach ungesättigte langkettige Fettsäuren: 0,01 bis 2 g,
zwischen 10 mg und 6000 mg einer Kombination von aus Gerstenmalz und Bockshornklee oder Gerstenmalz und grünem Anis oder Bockshornklee und grünem Anis oder zusätzlich aus Gerstenmalz, Bockshornklee und grünem Anis ausgewählten Pflanzenextrakten
zusammengesetzt ist.

9. Dosis nach Anspruch 8,
**dadurch gekennzeichnet, dass**
sie zwischen 10 und 300 mg, insbesondere zwischen 170 und 210 mg Gestenmalz, zwischen 50 und 2000 mg, insbesondere zwischen 50 bis 150 mg Bockshornklee und zwischen 100 und 4000 mg, insbesondere 100 bis 200 mg grünen Anis enthält.

10. Zusammensetzung nach einem der Ansprüche 1 bis 7 zur Verwendung als Stimulanz und/oder Initiator der Laktation und/oder als Stimulanz des Saugens während des Stillens bei einem Säugetier, insbesondere beim Menschen.

11. Dosis nach einem der Ansprüche 8 bis 9 zur Verwendung als Stimulanz und/oder Initiator der Laktation und/oder als Stimulanz des Saugens während des Stillens bei einem Säugetier, insbesondere beim Menschen.

## Claims

1. Nutritional composition comprising:
- a protein fraction,
- a glucid fraction,
- a lipid fraction comprising at least one fatty acid in the omega-3 group,
- at least one mineral element,
- at least one vitamin including vitamin D,
- a combinaison of plant extracts, the combinaison beeing choosen from :
- an extract of barley malt plant (Hordeum vulgare), fenugreek (Trigonella foenum graecum) and anise (Pimpinella anisum), or
- an extract of barley malt plant (Hordeum vulgare) and fenugreek (Trigonella foenum graecum), or
- an extract of barley malt plant (Hordeum vulgare) and anise (Pimpinella anisum), or
- an extract of fenugreek (Trigonella foenum graecum) and anise (Pimpinella anisum).

2. Composition according to claim 1, **characterised in that** it comprises a milk base chosen from a liquid milk or a dried milk concentrate.

3. Composition according to one of claims 1 to 2, **characterised in that** the said fatty acid in the omega-3 group is docosahexaenoic acid or DHA.

4. Composition according to one of claims 1 to 3, **characterised in that** it comprises iodine, selenium and magnesium among the minerals.

5. Composition according to one of claims 1 to 4, **characterised in that** it also comprises a source of fibres, preferentially chosen from galactofructose and/or fructooligosaccharide.

6. Composition according to one of claims 1 to 5, **characterised in that** it is in the form of a liquid, a powder, a food, a tablet, a granule, a solution contained in an ampoule or any other suitable galenic form.

7. Composition as defined in one of claims 1 to 6 for its use as a medication.

8. Dose of a composition as defined in one of claims 1 to 6, **characterised in that** it is formed by:
Vitamin D: from 0.1 µg to 10 µg, in particular 1 to 2 µg,
Calcium: 100 mg to 1 g, in particular 200 to 300 mg,
Selenium: from 1 to 100 µg, in particular 10 to 30 µg,
Iodine: 10 to 200 µg, in particular 30 to 60 µg,
Magnesium: 10 to 400 mg, in particular 110 to 130 mg,
Phosphorus: 100 to 1000 mg, in particular 230 to 250 mg,
Fibres: 0.1 to 30 mg,
Docosahexaenoic acid: 10 to 500 mg, in particular 50 to 150 mg,
Long-chain polyunsaturated fatty acids: 0.01 to 2 g,
between 10 and 6000 mg of each plant extract of the combinaison chosen from :
- an extract of barley malt plant, fenugreek and anise, or
- an extract of barley malt plant and fenugreek , or
- an extract of barley malt plant and anise, or
- an extract of fenugreek and anise.

9. Dose according to claim 8, **characterised in that** it comprises between 10 and 300 mg, more particulary between 170 and 210 mg of barley malt, between 50 and 2000 mg, more particulary between 50 and 150 mg, of fenugreek, and between 100 and 4000 mg, more particulary between 100 and 200 mg of anise.

10. Composition as defined in one of claims 1 to 7 for its use to stimulate and/or initiate lactation and/or to stimulate suction during breast-feeding in a mammal.

11. Dose as defined in one of claims 8 to 9 for its use to stimulate and/or initiate lactation and/or to stimulate suction during breastfeeding
in a mammals.
